(19) Europäisches Patentamt
European Patent Office
Office européen des brevets

(11) **EP 3 799 793 B1**

(12) **EUROPEAN PATENT SPECIFICATION**

(45) Date of publication and mention
of the grant of the patent:
**28.09.2022 Bulletin 2022/39**

(21) Application number: **19814576.5**

(22) Date of filing: **05.06.2019**

(51) International Patent Classification (IPC):
***A61B 8/08*** *(2006.01)*      ***A61B 8/00*** *(2006.01)*

(52) Cooperative Patent Classification (CPC):
**A61B 8/4254; A61B 8/429; A61B 8/4455;
A61B 8/54;** A61B 8/4236

(86) International application number:
**PCT/JP2019/022325**

(87) International publication number:
**WO 2019/235521 (12.12.2019 Gazette 2019/50)**

(54) **ULTRASONIC MEASUREMENT DEVICE, CONTACT DETERMINATION SERVER DEVICE, CONTACT DETERMINATION PROGRAM, AND CONTACT DETERMINATION METHOD**

ULTRASCHALLMESSVORRICHTUNG, KONTAKTBESTIMMUNGSSERVERVORRICHTUNG, KONTAKTBESTIMMUNGSPROGRAMM UND KONTAKTBESTIMMUNGSVERFAHREN

DISPOSITIF DE MESURE ULTRASONORE, DISPOSITIF SERVEUR DE DÉTERMINATION DE CONTACT, PROGRAMME DE DÉTERMINATION DE CONTACT ET PROCÉDÉ DE DÉTERMINATION DE CONTACT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.06.2018 JP 2018109363**

(43) Date of publication of application:
**07.04.2021 Bulletin 2021/14**

(73) Proprietor: **Triple W Japan Inc.
Tokyo 105-0001 (JP)**

(72) Inventors:
• **KATAYAMA Yutaka
Tokyo 1050001 (JP)**
• **MASAMORI Ryosuke
Tokyo 1050001 (JP)**

(74) Representative: **Puschmann Borchert Kaiser
Klettner
Patentanwälte Partnerschaft mbB
Bajuwarenring 21
82041 Oberhaching (DE)**

(56) References cited:
WO-A1-2016/199182   JP-A- 2017 012 612
US-A1- 2009 105 585   US-A1- 2014 037 168
US-A1- 2017 258 386   US-A1- 2017 273 664
US-A1- 2017 319 077

**Description**

FIELD

The technique disclosed here relates to an ultrasonic measurement device, a contact determination server device, a contact determination program, and a contact determination method.

BACKGROUND

US 2014/037168 A1 discloses a contact determination server device communicable, through a network, with a terminal device including a probe, the probe being configured to be continuously attached to a body surface of a subject while being in contact with the body surface, transmit ultrasonic waves into a body of the subject, and receive reflected waves of ultrasonic waves, the contact determination server device being configured to receive reflected waves transmitted from the terminal device, the contact determination server device comprising a controller configured to determine a contact state of the probe with the body surface, wherein the controller determines the contact state of the probe with the body surface by comparing at least two received waves acquired through transmission and reception of ultrasonic waves by the probe at different times.

Patent Document 1 discloses a device that brings a probe into contact with the body surface of a subject so that the probe transmits ultrasonic waves into the body and receives reflected waves of the ultrasonic waves. The device described in Patent Document 1 estimates a urine volume in the urinary bladder based on the received reflected waves.

CITATION LIST

PATENT DOCUMENT

Patent Document 1: Japanese Patent Application Publication No. 2016-43274

SUMMARY

TECHNICAL PROBLEM

In such a device, the probe needs to be appropriately brought into contact with the body surface. For example, in a situation where an operator presses a probe against the body surface to acquire reflected waves from the probe, the operator can acquire reflected waves while appropriately adjusting a contact state of the probe. In the state where the probe is kept attached, however, the contact state of the probe cannot be adjusted as necessary. In such a case, the contact state of the probe with the body surface needs to be determined appropriately. If the contact state is inappropriate, the probe is attached again, or reflected waves from the probe at this time are not used, for example.

It is therefore an object of the technique disclosed here to determine a contact state of a probe with a body surface.

SOLUTION TO PROBLEM

The invention is defined in the independent claims. Further aspects are defined in the dependent claims.

ADVANTAGES OF INVENTION

The ultrasonic measurement device disclosed here is capable of determining a contact state of a probe with a body surface.

The contact determination server device disclosed here is capable of determining a contact state of a probe with a body surface.

The contact determination program disclosed here is capable of determining a contact state of a probe with a body surface.

The contact determination method disclosed here is capable of determining a contact state of a probe with a body surface.

BRIEF DESCRIPTION OF THE DRAWINGS

[FIG. 1] FIG. 1 is a schematic view of an ultrasonic measurement device.

[FIG. 2] FIG. 2 is a schematic perspective view of a probe.

[FIG. 3] FIG. 3 is a schematic side view of the probe.

[FIG. 4] FIG. 4 is a view illustrating an attached state of the probe.

[FIG. 5] FIG. 5 is a schematic cross-sectional view of lower abdomen of a human body to which the probe is attached.

[FIG. 6] FIG. 6 is a block diagram of a terminal device.

[FIG. 7] FIG. 7 is a block diagram illustrating a hardware configuration of a first server.

[FIG. 8] FIG. 8 is a block diagram illustrating a function configuration of a controller.

[FIG. 9] FIG. 9 is a flowchart depicting a process of an ultrasonic measurement device.

[FIG. 10] FIG. 10 is a flowchart of a subroutine of urine volume estimation.

[FIG. 11] FIG. 11 is a schematic cross-sectional view of lower abdomen of a human body with approximately a middle urine accumulation volume.

[FIG. 12] FIG. 12 shows a received signal of a first ultrasonic sensor in the state of FIG. 11.

[FIG. 13] FIG. 13 shows a received signal of a second ultrasonic sensor in the state of FIG. 11.

[FIG. 14] FIG. 14 shows a received signal of a third ultrasonic sensor in the state of FIG. 11.

[FIG. 15] FIG. 15 shows a received signal of a fourth ultrasonic sensor in the state of FIG. 11.

[FIG. 16] FIG. 16 is a table showing a determination result stored in a third server.

[FIG. 17] FIG. 17 is a flowchart of a subroutine of contact determination.

[FIG. 18] FIG. 18 shows a first latest received signal of the third ultrasonic sensor in an appropriate contact state of the probe with a body surface.

[FIG. 19] FIG. 19 shows a second latest received waveform of the third ultrasonic sensor in the appropriate contact state of the probe with the body surface.

[FIG. 20] FIG. 20 shows a third latest received waveform of the third ultrasonic sensor in the appropriate contact state of the probe with the body surface.

[FIG. 21] FIG. 21 is a first latest received signal of the third ultrasonic sensor in an inappropriate contact state of the probe with the body surface.

[FIG. 22] FIG. 22 is a second latest received waveform of the third ultrasonic sensor in the inappropriate contact state of the probe with the body surface.

[FIG. 23] FIG. 23 is a third latest received waveform of the third ultrasonic sensor in the inappropriate contact state of the probe with the body surface.

[FIG. 24] FIG. 24 is a block diagram illustrating a hardware configuration of a user terminal according to another embodiment.

## DESCRIPTION OF EMBODIMENTS

An exemplary embodiment will be described in detail hereinafter with reference to the drawings.

<Configuration of Ultrasonic Measurement Device>

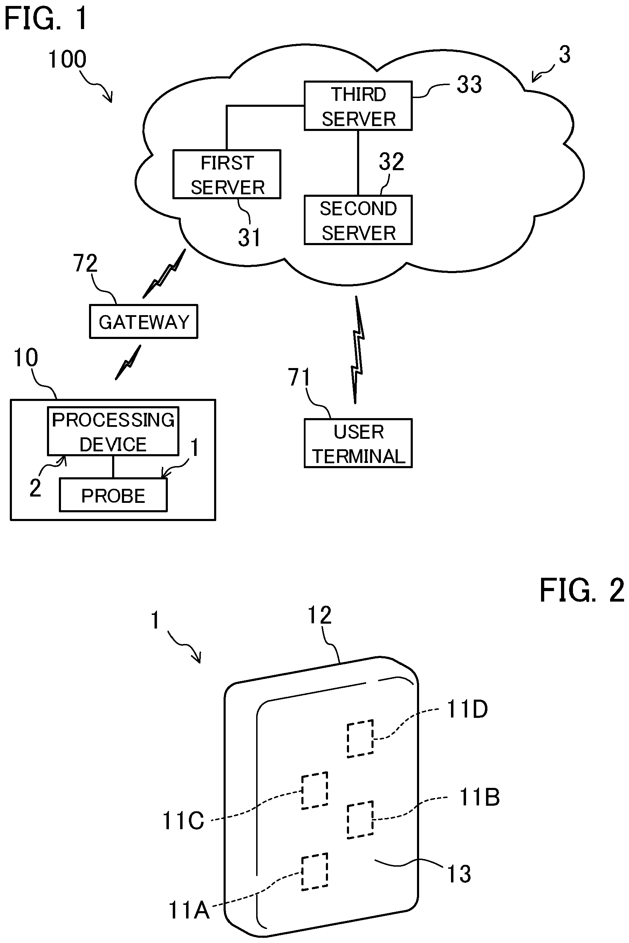

FIG. 1 is a schematic view of an ultrasonic measurement device 100.

The ultrasonic measurement device 100 estimates a urination timing of a subject using ultrasonic waves. Examples of the subject include those who require care, such as elderly people and physically disabled people, and those who do not require care but have difficulty in moving and need time for going to the lavatory. The subject, however, is not limited to these examples.

The ultrasonic measurement device 100 includes a terminal device 10 and a server group 3. The terminal device 10 includes: a probe 1 configured to be attached to a body surface of the subject while being in contact with the body surface, transmit ultrasonic waves into the body, and receive reflected waves of ultrasonic waves; and a processing device 2 configured to process a received signal received by the probe 1. The server group 3 analyzes a signal transmitted from the terminal device 10. The probe 1 is continuously attached to the subject, and repeatedly transmits and receives ultrasonic waves. The probe 1 and the processing device 2 are connected to each other by wires. The processing device 2 wirelessly communicates with the server group 3.

<Probe>

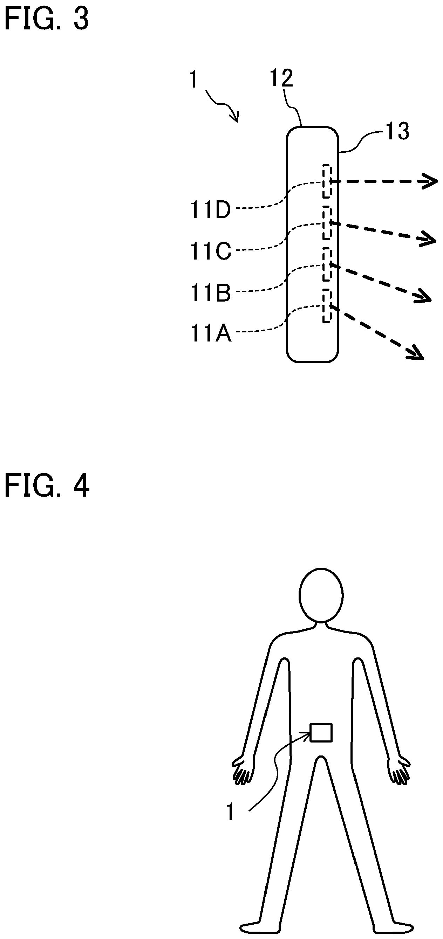

FIG. 2 is a schematic perspective view of the probe 1. FIG. 3 is a schematic side view of the probe 1.

As illustrated in FIG. 2, the probe 1 includes four ultrasonic sensors 11A through 11D and a casing 12 for housing the ultrasonic sensors 11A through 11D.

The ultrasonic sensors 11A through 11D have the same basic configuration. Hereinafter, each of the ultrasonic sensors 11A through 11D will be simply referred to as an "ultrasonic sensor 11" when not being distinguished from one another. When the ultrasonic sensors 11A through 11D are distinguished from one another, the ultrasonic sensors 11A through 11D will be referred to as a first ultrasonic sensor 11A, a second ultrasonic sensor 11B, a third ultrasonic sensor 11C, and a fourth ultrasonic sensor 11D, respectively.

The ultrasonic sensor 11 transmits and receives ultrasonic waves. Specifically, the ultrasonic sensor 11 includes a piezoelectric element. The piezoelectric element generates ultrasonic waves by generating vibrations in accordance with a driving voltage, whereas when receiving ultrasonic waves, the piezoelectric element generates an electrical signal in accordance with the vibrations thereof.

As illustrated in FIG. 2, the casing 12 is generally formed in a flat rectangular parallelepiped. The casing 12 has a pair of opposed substantially rectangular surfaces each having a relatively large area, one of the pair of opposed substantially rectangular surfaces serves as a surface to be brought into contact with the abdomen of a subject (hereinafter referred to as a "contact surface") 13.

The ultrasonic sensors 11A through 11D are arranged to transmit ultrasonic waves toward different positions in a direction in which the urinary bladder expands.

Specifically, as illustrated in FIGS. 2 and 3, the ultrasonic sensors 11A through 11D are arranged at different positions in the vertical direction of the casing 12. The first ultrasonic sensor 11A, the second ultrasonic sensor 11B, the third ultrasonic sensor 11C, and the fourth ultrasonic sensor 11D are arranged in this order from below. Here, the vertical direction (top-bottom direction) of the casing 12 is a vertical direction when the probe 1 is attached to the subject. The probe 1 is attached to the subject in a state where the longitudinal direction of the substantially rectangular contact surface 13 coincides with the vertical direction, for example. That is, the vertical direction of the casing 12 is the longitudinal direction of the contact surface 13.

The positions of the first ultrasonic sensor 11A and the third ultrasonic sensor 11C are the same in the lateral direction (left-right direction), whereas the second ultrasonic sensor and the fourth ultrasonic sensor 11D are offset from the first ultrasonic sensor 11A and the third ultrasonic sensor 11C in the lateral direction. The positions of the second ultrasonic sensor 11C and the fourth ultrasonic sensor 11D are the same in the lateral direction. That is, the ultrasonic sensors 11A through 11D are arranged in a staggered pattern.

In addition, transmission direction of ultrasonic waves from the ultrasonic sensors 11A through 11D are not parallel. As illustrated in FIG. 3, the ultrasonic sensors 11A through 11D radially transmit ultrasonic waves in the vertical direction. That is, the ultrasonic sensors 11A through 11D emit ultrasonic waves at different angles in the vertical direction. Specifically, the fourth ultrasonic sensor 11D transmits ultrasonic waves in the direction normal to the contact surface 13. The third ultrasonic sensor 11C transmits ultrasonic waves to a direction obliquely below the transmission direction of ultrasonic waves from the fourth ultrasonic sensor 11A. The second ultrasonic sensor 11B transmits ultrasonic waves to a direction obliquely below the transmission direction of ultrasonic waves from the third ultrasonic sensor 11C. The first ultrasonic sensor 11A transmits ultrasonic waves obliquely downward from the transmission direction of ultrasonic waves from the second ultrasonic sensor 11B. That is, the first ultrasonic sensor 11A transmits ultrasonic waves in the most downward direction, and the direction of transmission of ultrasonic waves gradually turns upward in the order of the second ultrasonic sensor 11B, the third ultrasonic sensor 11C, and the fourth ultrasonic sensor 11D.

FIG. 4 is a view illustrating an attached state of the probe 1. As illustrated in FIG. 4, the probe 1 is continuously attached to the subject. The probe 1 is disposed on a portion of the skin of the abdomen of the subject corresponding to the urinary bladder (e.g., the lower abdomen).

For example, the probe 1 is attached to the abdomen of the subject with a belt or a tape with the contact surface 13 being in contact with the abdomen. Gel or the like for increasing permeability of ultrasonic waves to the abdomen is applied between the contact surface 13 and the abdomen.

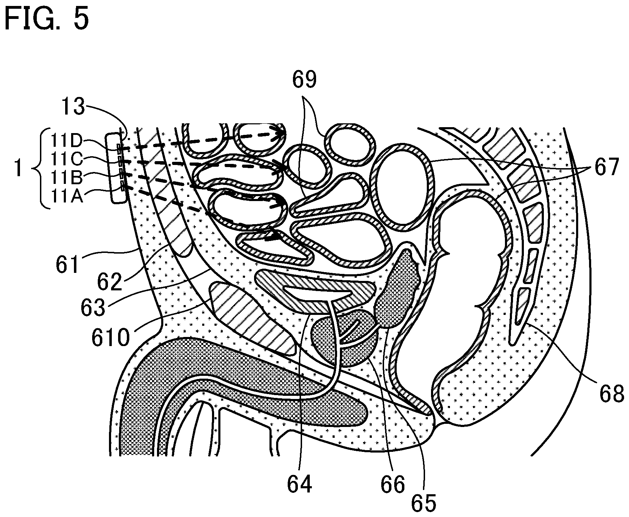

FIG. 5 is a schematic cross-sectional view of the lower abdomen of a human body to which the probe 1 is attached. The example of FIG. 5 shows a state where substantially no urine is accumulated. Subcutaneous fat 61, muscle 62, fat 63, urinary bladder 64, seminal vesicle 65 or prostate 66 (in a male) or vagina (in a female), rectum 67, backbone (sacrum) 68, and others are arranged in this order from the surface of the abdomen toward the back. Small intestine 69 is located above the urinary bladder 64, and pubis 610 is located obliquely below the front of the urinary bladder 64.

The probe 1 is attached to the abdomen of the subject such that ultrasonic waves emitted from the ultrasonic sensors 11A through 11D expand in the vertical direction. The ultrasonic sensors 11A through 11D transmit ultrasonic waves toward different positions in the vertical direction in the body. The urinary bladder expands in three dimensions with an increase in urine accumulation volume. Thus, the different positions in the vertical direction are one of directions in which the urinary bladder expands. The urinary bladder greatly expands especially in the vertical direction. That is, the ultrasonic sensors 11A through 11D are arranged to transmit ultrasonic waves toward different positions in the direction in which the urinary bladder expands relatively greatly.

<Processing Device>

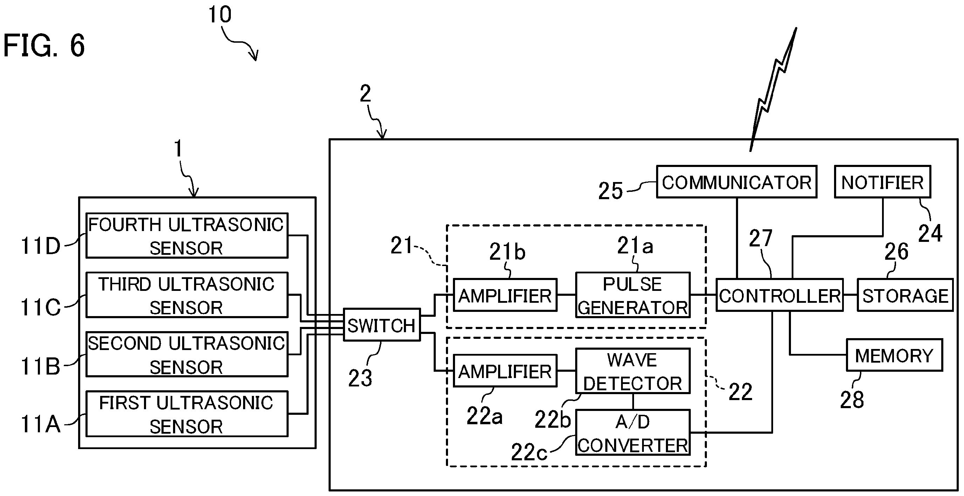

FIG. 6 is a block diagram of the terminal device 10. The processing device 2 includes a transmitter 21 that outputs a driving voltage to the ultrasonic sensors 11, a receiver 22 that receives an electrical signal from the ultrasonic sensors 11, a switch 23 that sequentially selects one of the ultrasonic sensors 11 to be connected to the transmitter 21 and the receiver 22, a notifier 24 that notifies an external device of various types of information, a communicator 25 that communicates with an external device, a storage 26 that stores various programs and data, a controller 27 that controls the entire processing device 2, and a memory 28. The processing device 2 is attached to, for example, a cloth of the subject.

The transmitter 21 supplies a driving voltage to the ultrasonic sensor 11. The transmitter 21 includes a pulse generator 21a and an amplifier 21b. The pulse generator 21a generates a pulse signal having a predetermined pulse width and a predetermined voltage value. The pulse generator 21a may be configured such that the pulse width, the number of pulses, and the frequency are changeable. The amplifier 21b amplifies a pulse signal from the pulse generator 21a, and outputs the amplified pulse signal to the ultrasonic sensor 11 as a driving voltage.

The receiver 22 receives the electrical signal from the ultrasonic sensor 11. The receiver 22 includes the amplifier 22a, a wave detector 22b, and an analog-to-digital (A/D) converter 22c. The amplifier 22a amplifies the received signal from the ultrasonic sensor 11. The wave detector 22b performs envelope detection on the amplified received signal. Note that the wave detector 22b may amplify the received signal after the detection. The A/D converter 22c performs A/D conversion on the received signal after the detection.

The switch 23 selects one of the ultrasonic sensors 11A through 11D as the ultrasonic sensor 11 to be connected to the transmitter 21 and the receiver 22.

The notifier 24 is, for example, an LED lamp. The LED lamp notifies the subject of various types of information (e.g., arrival of a urination timing) by using an illumination mode of the LED lamp.

The communicator 25 is a communication module, and communicates with an external communication device. For example, the communicator 25 performs communication in Bluetooth (registered trademark) standard. As illustrated in FIG. 1, the communicator 25 communicates with the server group 3 through a gateway 72.

The storage 26 is a computer-readable recording medium, and is, for example, a flash memory. Note that the storage 26 may be, for example, an optical disk such as a CD-ROM. The storage 26 stores various programs and various types of information necessary for executing processing of the controller 27. The storage 26 also stores a received signal received by the receiver 22 and information acquired from the outside through the communicator 25, for example.

The controller 27 controls the transmitter 21, the receiver 22, the switch 23, the notifier 24, and the communicator 25 based on programs stored in the storage 26. The controller 27 is a processor such as a central processing unit (CPU). The controller 27 executes various types of processing by developing programs stored in, for example, the storage 26 to the memory 28 and executing the developed programs. Note that the controller 27 may be implemented by hardware such as large scale integration (LSI) having functions similar to those of a processor.

Specifically, the controller 27 controls the switch 23 to select one of the ultrasonic sensors 11 to be connected to the transmitter 21 and the receiver 22. The controller 27 controls the transmitter 21 such that the transmitter 21 outputs a driving voltage to the ultrasonic sensor 11. The controller 27 controls the receiver 22 such that the receiver 22 converts the received signal received by the ultrasonic sensor 11 to a digital signal. The controller 27 controls the communicator 25 such that the communicator 25 transmits the signal from the receiver 22 to the outside. The controller 27 receives a signal from the outside through the communicator 25 and performs processing in accordance with the signal (e.g., operates the notifier 24).

The memory 28 is a computer-readable recording medium, and is, for example, a read only memory (ROM), an erasable programmable ROM (EPROM), an electrically erasable programmable ROM (EEPROM), or a random access memory (RAM).

<Server Group>

The server group 3 performs so-called cloud computing. As illustrated in FIG. 1, the server group 3 includes a plurality of servers. Specifically, the server group 3 includes a first server 31 that performs data analysis, a second server 32 that provides a user terminal 71 with an application, and a third server 33 that functions as a database.

The first server 31 is capable of communicating with the terminal device 10 (specifically, the processing device 2) through a network, receives a received signal transmitted from the terminal device 10 (i.e., a received signal (received wave) of the ultrasonic sensor 11 subjected to reception processing by the receiver 22), and stores the received signal in the third server 33. The received signal transmitted from the terminal device 10 will be hereinafter also simply referred to as a "received signal of the ultrasonic sensor 11." The first server 31 analyzes the received signal of the ultrasonic sensor 11 stored in the third server 33. Specifically, the first server 31 estimates a urine accumulation volume in the urinary bladder based on the received signal of the ultrasonic sensor 11. In addition, the first server 31 determines a contact state of the probe 1 with the body surface based on the received signal of the ultrasonic sensor 11. The first server 31 stores, for example, programs and data for estimating a urine accumulation volume and programs and data for determining the contact state of the probe 1. The first server 31 is an example of a contact determination server device.

The second server 32 is capable of communicating with the user terminal 71 through the network. The server group 3 (specifically, the third server 33) is capable of registering a user (user ID), and the user ID is stored in the server group 3 in association with the processing device 2. The server group 3 is also capable of registering a user terminal 71 in association with the user ID. For example, when a user performs user registration or a login to the second server 32 by using the user terminal 71, the second server 32 acquires and registers information on the user terminal 71. Accordingly, the server group 3 and the user terminal 71 are allowed to communicate with each other. For example, a user terminal 71 (e.g., a smartphone or a tablet terminal) carried by a caregiver can be registered in the server group 3. If a subject is capable of going to the lavatory by himself or herself, a user terminal 71 of the subject can be registered. The number of user terminals 71 is not limited to one, and a plurality of user terminals (e.g., the user terminal 71 of the caregiver and the user terminal 71 of the subject) may be registered. An application dedicated to the ultrasonic measurement device 100 may be downloaded in the user terminal 71 so that the dedicated application can be operated by transmitting or receiving information to/from the second server 32. The second server 32 transmits various types of information such as a urination timing of the subject to the user terminal 71.

The third server 33 stores information on the subject, the received signal of the ultrasonic sensor 11 received by the first server 31, an analysis result of the first server 31, and information received by the second server 32. Examples of the information on the subject include a user ID for specifying the subject, a device ID for specifying the processing device 2, a terminal ID for specifying a user terminal, and information on urine accumulation and urination of the subject. The third server 33 stores these types of information in association with one another. The user ID, the device ID, and the terminal ID are registered beforehand by the user. The information on urine accumulation and urination of the subject is, for example, an allowable urine accumulation volume (allowable urine level described later), and a common initial value is set beforehand as a default value.

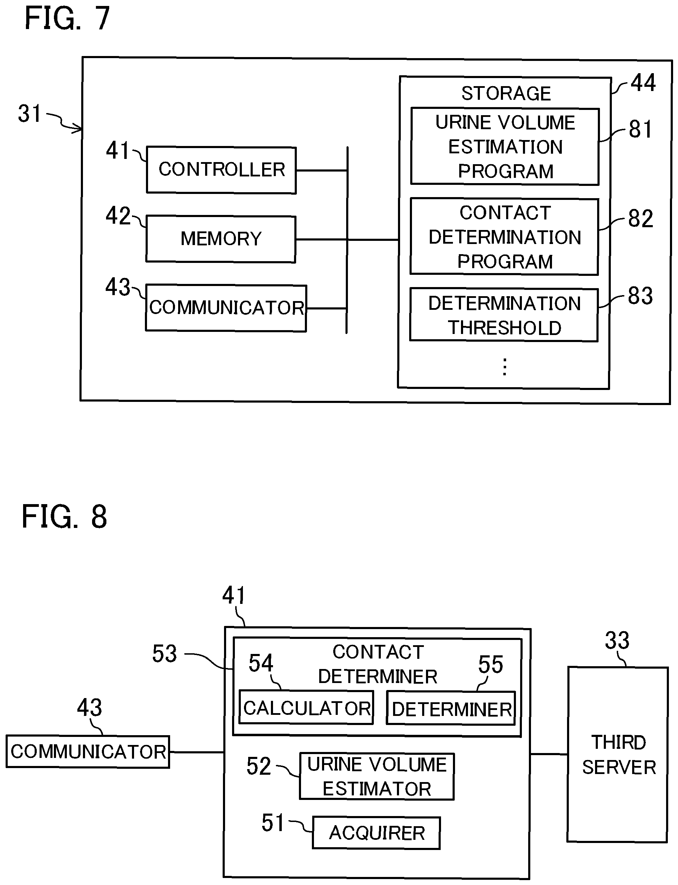

FIG. 7 is a block diagram illustrating a hardware configuration of the first server 31. The first server 31 includes a controller 41, a memory 42, a communicator 43, and a storage 44. Note that the first server 31 may further include a keyboard and/or a display.

The controller 41 is, for example, a processor such as a central processing unit (CPU). The controller 41 executes various processing by developing programs stored in, for example, the storage 44 to the memory 42 and executing the developed programs. Note that the controller 41 may be implemented by hardware such as large scale integration (LSI) having similar configurations to a processor.

The memory 42 is a computer-readable recording medium, and is, for example, a read only memory (ROM), an erasable programmable ROM (EPROM), an electrically erasable programmable ROM (EEPROM), or a random access memory (RAM).

The communicator 43 is a communication module, and communicates with the terminal device 10 through the gateway 72.

The storage 44 is a computer-readable recording medium, and is, for example, a hard disk. Note that the storage 44 may be, for example, an optical disk such as a CD-ROM. The storage 44 stores programs and various types of information necessary for executing processing of the controller 41. For example, the storage 44 stores a urine volume estimation program 81, a contact determination program 82, and a determination threshold 83 to be used for contact determination, for example.

The second server 32 and the third server 33 have basically the same hardware configuration as the first server 31. The programs and various types of information stored in the storages of the second server 32 and the third server 33 are programs and information in accordance with processing for the servers.

FIG. 8 is a block diagram illustrating a function configuration of the controller 41. The controller 41 includes an acquirer 51 that acquires a received signal of the ultrasonic sensor 11 by communication with the terminal device 10, a urine volume estimator 52 that estimates a urine accumulation volume in the urinary bladder of the subject, and a contact determiner 53 that determines a contact state of the probe 1 with the body surface.

The acquirer 51 receives a received signal of the ultrasonic sensor 11 transmitted from the processing device 2 of the terminal device 10. This acquisition function is implemented by execution of an associated program stored in the storage 44 by the controller 41. The acquirer 51 stores the received signal in the third server 33 together with a time at which the received signal is received (i.e., an acquisition time). The third server 33 accumulates received signals and acquisition times.

The urine volume estimator 52 estimates a urine accumulation volume in the urinary bladder based on reflected waves included in the received signal of the ultrasonic sensor 11. This estimation function is implemented by executing the urine volume estimation program 81 stored in the storage 44 by the controller 41. Specifically, the urine volume estimator 52 analyzes the received signal of the ultrasonic sensor 11 stored in the third server 33 and estimates a urine accumulation volume. The urine volume estimator 52 stores the estimation result in the third server 33. The urine volume estimator 52 transmits the estimation result to the processing device 2 and the user terminal 71 through the communicator 43 as necessary.

The contact determiner 53 determines a contact state of the probe 1 with the body surface by comparing at least two received signals acquired through transmission and reception of ultrasonic waves by the probe 1 at different times. More specifically, the contact determiner 53 determines a contact state of the probe 1 with the body surface based on the degree of similarity between at least two received signals acquired at different times. The contact determiner 53 determines that the contact state of the probe 1 is inappropriate if the degree of similarity is higher than a predetermined criterion, that is, two received signals are similar to each other, and determines that the contact state of the probe 1 is appropriate if the degree of similarity is lower than the predetermined criterion, that is, two received signals are not similar to each other. The contact determiner 53 includes a calculator 54 that calculates a similarity between two received signals, and a determiner 55 that determines a contact state based on the similarity. The "similarity" is an index indicating the degree of similarity. As the similarity increases, two received signals are more similar to each other. This determination function, specifically, the calculation function and the determination function, is implemented by executing the contact determination program 82 stored in the storage 44 by the controller 41.

The calculator 54 calculates similarities among received signals, that is, received waves, of a plurality of ultrasonic sensors 11 acquired at different times.

If a similarity is large, the determiner 55 determines that the contact state of the probe 1 with the body surface is inappropriate. On the other hand, if the similarity is small, the determiner 55 determines that the contact state of the probe 1 with the body surface is appropriate. The determiner 55 stores the determination result in the third server 33. The determiner 55 transmits the determination result to the processing device 2 and the user terminal 71 as necessary.

<Operation of Ultrasonic Measurement Device>

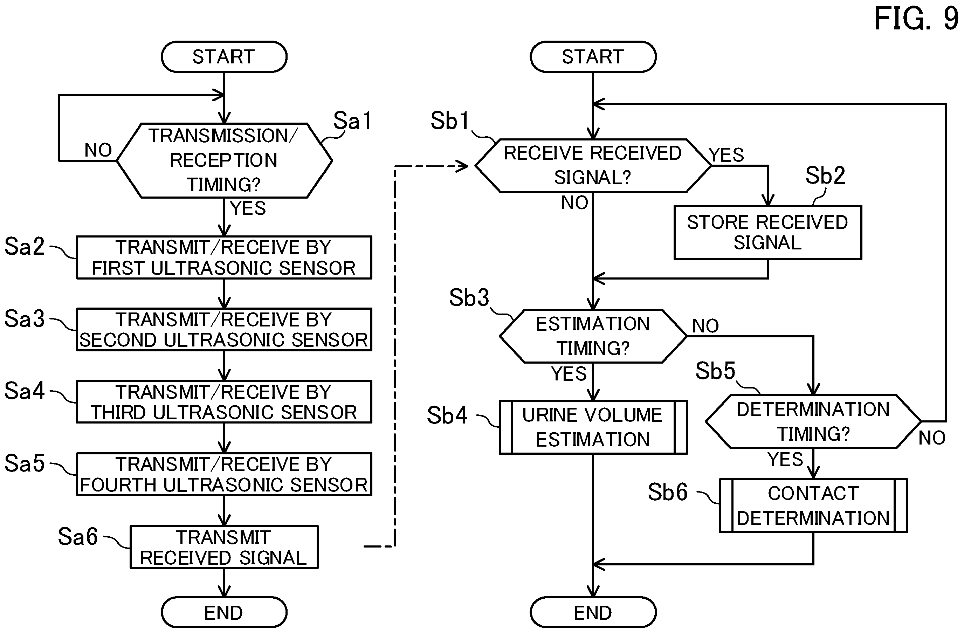

A process of the ultrasonic measurement device 100 will be hereinafter described in detail. FIG. 9 is a flowchart depicting a process of the ultrasonic measurement device 100.

First, operations of the probe 1 and the processing device 2 will be described. The probe 1 and the processing device 2 periodically transmit and receive ultrasonic waves. These transmission and reception of ultrasonic waves are controlled by the controller 27 of the processing device 2.

Specifically, in step Sa1, the controller 27 determines whether a transmission/reception timing has arrived or not. The transmission/reception timing is repeated in predetermined transmission/reception cycles. If the transmission/reception timing has not arrived yet, the controller 27 waits for the transmission/reception timing in step Sa1.

If the transmission/reception timing has arrived, the controller 27 causes the first through fourth ultrasonic sensors 11A through 11D to sequentially transmit and receive ultrasonic waves in order with switching by the switch 23. Specifically, in step Sa2, the controller 27 controls the switch 23 such that the first ultrasonic sensor 11A is connected to the transmitter 21 and the receiver 22. Then, the controller 27 outputs an instruction of generating a pulse signal to the transmitter 21, and causes the transmitter 21 to supply a driving voltage to the first ultrasonic sensor 11A. The first ultrasonic sensor 11A transmits ultrasonic waves based on the driving voltage, and receives reflected waves from the inside of the body. The received signal of the first ultrasonic sensor 11A is amplified and subjected to wave detection and A/D conversion by the receiver 22. The controller 27 stores the received signal after the A/D conversion in the storage 26. Thereafter, in steps Sa3 through Sa5, the controller 27 sequentially operates the switch 23 to perform similar control on the second through fourth ultrasonic sensors 11B through 11D.

Subsequently, in step Sa6, the controller 27 transmits received signals of the first through fourth ultrasonic sensors 11A through 11D to the server group 3 (specifically, the first server 31) through the communicator 25.

The controller 27 repeats the process from step Sa1. Specifically, the controller 27 periodically performs a set of processes in transmission/reception cycles. This set of processes includes transmission and reception of ultrasonic waves by the first through fourth ultrasonic sensors 11A through 11D and transmission of the received signals to the server group 3. These processes in steps Sa1 through Sa5 are an example of an acquisition step.

Next, a process of the server group 3 will be described.

In step Sb1, the acquirer 51 of the first server 31 determines whether four received signals of the ultrasonic sensors 11 periodically transmitted from the processing device 2 are received or not. If the received signals of the ultrasonic sensors 11 are received, the acquirer 51 stores the received signals of the ultrasonic sensors 11 in the third server 33 in step Sb2.

If the acquirer 51 does not receive received signals of the ultrasonic sensors 11, the urine volume estimator 52 determines whether an estimation timing has arrived or not in step Sb3. The estimation timing is repeated in predetermined estimation cycles. That is, the urine volume estimator 52 is configured to estimate a urine volume in estimation cycles. The duration of the estimation cycle is set to be longer than that of the transmission/reception cycle of the processing device 2. Note that the duration of the estimation cycle may be the same as that of the transmission/reception cycle.

If the estimation timing has arrived, the urine volume estimator 52 executes urine volume estimation in step Sb4. The urine volume estimation will be specifically described later. After the urine volume estimation has been completed, the acquirer 51 receives received signals of the ultrasonic sensors 11 again in step Sb1.

If the estimation timing has not arrived, the contact determiner 53 determines whether a determination timing has arrived or not in step Sb5. The determination timing is repeated in predetermined determination cycles. That is, the contact determiner 53 is configured to determine a contact state of the probe 1 with the body surface in determination cycles. The duration of the determination cycle is set to be the same duration of the estimation cycle. Note that the duration of the determination cycle may be longer than that of the estimation cycle or shorter than that of the estimation cycle.

If the determination timing has arrived, the contact determiner 53 performs contact determination in step Sb6. The contact determination will be specifically described later. After the contact determination has been completed, the acquirer 51 receives received signals of the ultrasonic sensors 11 again in step Sb1.

If the determination timing has not arrived, the process returns to the process of the acquirer 51 in step Sb1.

<Urine Volume Estimation>

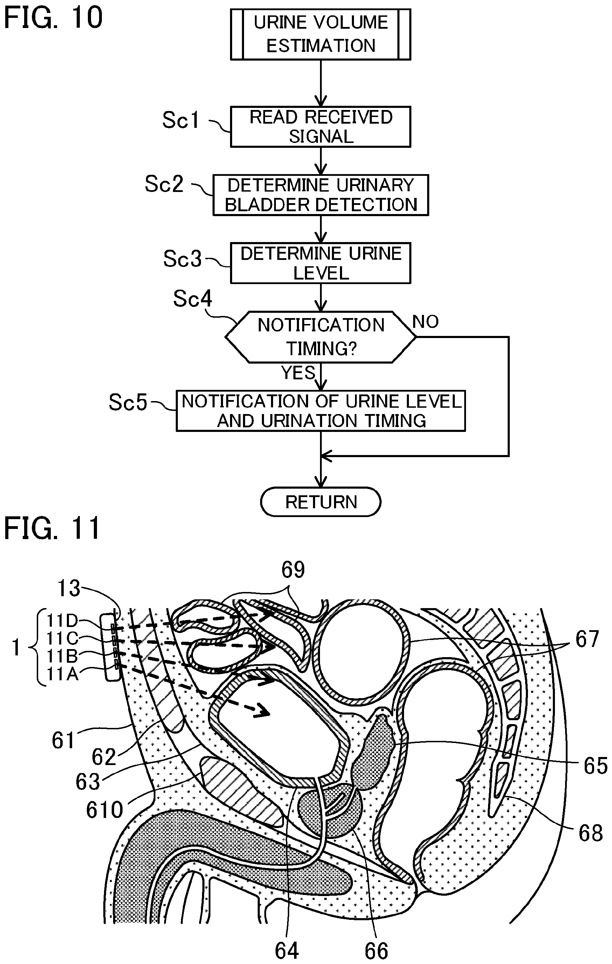

The urine volume estimation will now be specifically described. FIG. 10 is a flowchart of a subroutine of urine volume estimation.

In step Sc1, the urine volume estimator 52 reads out received signals of the ultrasonic sensors 11 from the third server 33. Since the probe 1 includes the four ultrasonic sensors 11, received signals of the ultrasonic sensors 11 to be read out refer to a set of four received signals. The urine volume estimator 52 reads out a predetermined number of latest sets (e.g., 10 sets) of received signals from the third server 33.

Next, in step Sc2, the urine volume estimator 52 determines whether the ultrasonic sensors 11 detect the urinary bladder or not based on received signals of the ultrasonic sensors 11. The urine volume estimator 52 examines whether the urinary bladder is detected or not for each of the four ultrasonic sensors 11.

Specifically, the urine volume estimator 52 performs an averaging process on each of the four received signals of the ultrasonic sensors 11. The urine volume estimator 52 averages the plurality of sets of read-out received signals for each ultrasonic sensor 11. Accordingly, noise of received signals can be reduced so that reflected waves can be easily identified. Thereafter, the urine volume estimator 52 determines whether the received signals after the averaging process include reflected waves of the urinary bladder. In the received signals, noise is observed immediately after transmission of ultrasonic waves, and thus, reflected waves from the posterior wall of the urinary bladder, which is relatively far from the surface of the abdomen, are easily identified. In view of this, the urine volume estimator 52 examines whether the received signals include reflected waves from the posterior wall of the urinary bladder or not. Since a reception time zone in which reflected waves from the posterior wall of the urinary bladder returns is generally known, the urine volume estimator 52 determines whether reflected waves are present in the reception time zone or not. The term "reflected waves from the urinary bladder" refers to reflected waves from the posterior wall of the urinary bladder, unless otherwise specified. The urine volume estimator 52 determines that the ultrasonic sensor 11 detects the urinary bladder based on the fact that received signals include reflected waves from the urinary bladder.

Subsequently, in step Sc3, the urine volume estimator 52 determines a urine level. The urine level is an index indicating a urine accumulation volume in the urinary bladder, and a higher urine level means a larger urine accumulation volume.

Specifically, the urine volume estimator 52 obtains a urine level based on which ultrasonic sensor 11 detects the urinary bladder. The urinary bladder expands upward with an increase in the urine accumulation volume, whereas the first through fourth ultrasonic sensors 11A through 11D transmit ultrasonic waves toward different positions in the vertical direction as described above. Thus, as the urine accumulation volume increases, the number of ultrasonic sensors 11 that detect the urinary bladder increases. The urine volume estimator 52 determines a urine level based on to which successive ultrasonic sensor 11 from the bottom detects the urinary bladder. It is assumed that the urine level is "0" if none of the ultrasonic sensors 11 detects the urinary bladder. The urine level is "1" if only the first ultrasonic sensor 11A detects the urinary bladder. The urine level is "2" if only the first ultrasonic sensor 11A and the second ultrasonic sensor 11B detect the urinary bladder. The urine level is "3" if only the first ultrasonic sensor 11A, the second ultrasonic sensor 11B, and the third ultrasonic sensor 11C detect the urinary bladder. The urine level is "4" if all the ultrasonic sensors 11 detect the urinary bladder.

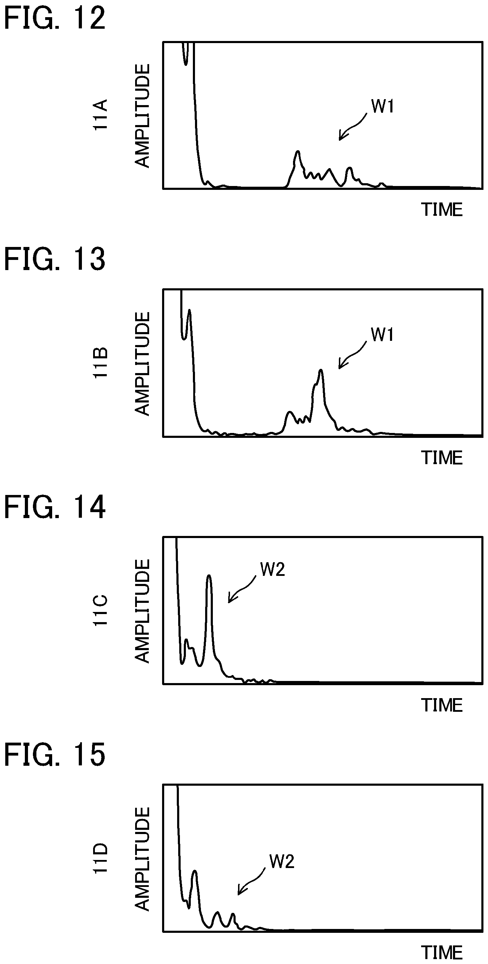

FIG. 11 illustrates an example of lower abdomen of a human body. FIG. 11 is a schematic cross-sectional view of lower abdomen of a human body with a middle urine accumulation volume. FIGS. 12 through15 illustrate examples of received signals of the four ultrasonic sensors 11 in the state of the urinary bladder shown in FIG. 11. FIG. 12 shows a received signal of the first ultrasonic sensor 11A. FIG. 13 shows a received signal of the second ultrasonic sensor 11B. FIG. 14 shows a received signal of the third ultrasonic sensor 11C. FIG. 15 shows a received signal of the fourth ultrasonic sensor 11D. The waveforms of FIGS. 12 through 15 are waveforms after being subjected to the averaging process.

In this example, the first ultrasonic sensor 11A and the second ultrasonic sensor 11B detect a reflected wave W1 of the urinary bladder, and the third ultrasonic sensor 11C and the fourth ultrasonic sensor 11D do not detect reflected waves from the urinary bladder. That is, the urine level is "2."

As illustrated in FIG. 11, the small intestine 69 is located above the urinary bladder 64. A large part of ultrasonic waves emitted from the third ultrasonic sensor 11C and the fourth ultrasonic sensor 11D enter the small intestine 69. Since gas is mixed in the small intestine 69, ultrasonic waves attenuate in the small intestine 69, and do not easily reach a deep portion in the body. Thus, in received signals of the third ultrasonic sensor 11C and the fourth ultrasonic sensor 11D (see FIGS. 14 and 15), although a reflected wave W2 of the small intestine is observed, not only reflected waves from the urinary bladder but also noticeable reflected waves from other portions are hardly observed, after the reflected wave W2 of the small intestine. Since the small intestine 69 always performs peristalsis, the reflected wave W2 from the small intestine does not appear as a clear peak, and may have a waveform similar to that of noise (see FIG. 15). With some degree of expansion of the urinary bladder 64, the small intestine 69 and the urinary bladder 64 are present on a propagation path of ultrasonic waves in some cases. In such cases, a small part of the small intestine 69 is present on the propagation path of ultrasonic waves, and thus, part of ultrasonic waves reaches as far as the urinary bladder 64, and reflected waves from the urinary bladder can be observed. At this time, reflected waves from the small intestine and reflected waves from the urinary bladder can be observed.

Note that the urine level may be determined simply based on the number of ultrasonic sensors 11 that detect the urinary bladder.

On the other hand, in the example of FIG. 5, since the urine accumulation volume is small and the urinary bladder is small, none of the ultrasonic sensors 11 detects reflected waves from the urinary bladder. The urine level in this case is "0."

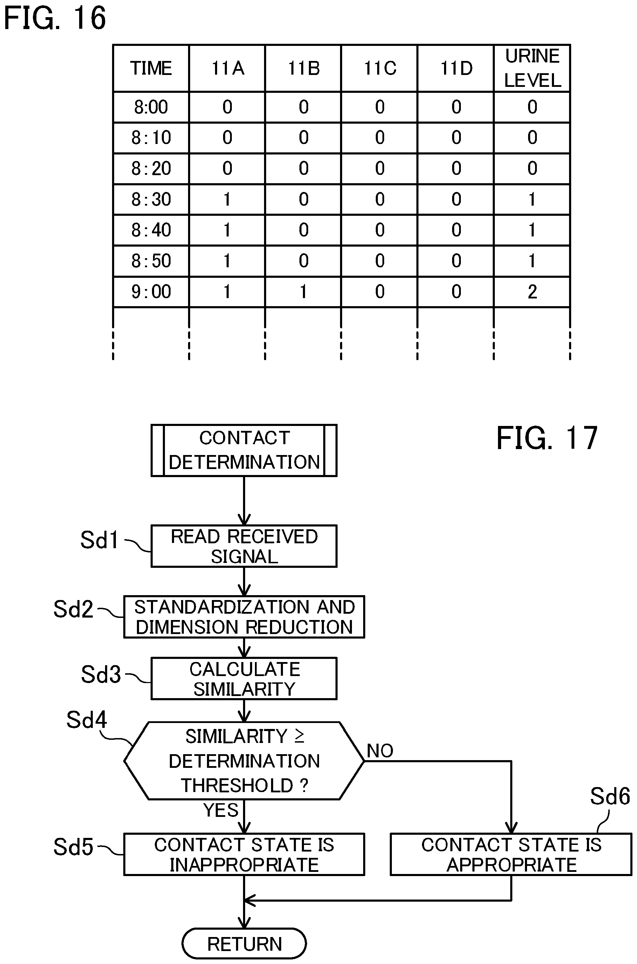

The urine volume estimator 52 causes the third server 33 to store the determination result. The determination result includes a determination time, the presence/absence of detection of the urinary bladder by each ultrasonic sensor 11, and the urine level. The determination results are accumulated in the third server 33 in the form of a table as shown in FIG. 16. Determination results at determination time 9:00 in FIG. 16 are results of received signals in FIGS. 12 through 15. Character "0" in the columns of the ultrasonic sensors 11 represents non-detection of the urinary bladder, and character "1" represents detection of the urinary bladder.

Thereafter, in step Sc4, the urine volume estimator 52 determines whether a notification timing has arrived or not. The notification timing is repeated in predetermined notification cycles. That is, the urine volume estimator 52 is configured to notify the processing device 2 of a urine volume in notification cycles. The duration of the notification cycle is set to be longer than that of the estimation cycle. Note that the duration of the notification cycle may be equal to that of the estimation cycle.

If the notification timing has not arrived, the urine volume estimator 52 finishes the urine volume estimation, and the process returns to the flowchart of FIG. 9 (specifically, step Sb4).

If the notification timing has arrived, the urine volume estimator 52 reads urine levels from the preceding notification timing to the current notification timing from the third server 33 in step Sc5. The urine volume estimator 52 transmits a maximum value of the read-out urine levels to the processing device 2 and the user terminal 71 through the communicator 43. The urine volume estimator 52 may transmit a mean value instead of the maximum value of the read-out urine levels. Alternatively, the urine volume estimator 52 may read a latest urine level stored in the third server 33 and transmit the latest urine level.

In this case, if the urine level to be notified has reached the allowable urine level, the urine volume estimator 52 transmits arrival of the urination timing to the processing device 2 and the user terminal 71 through the communicator 43. The allowable urine level is a urine level at which the subject has a urinary urge, and level "3", for example, is stored as an initial value in the storage 44.

On the other hand, when the processing device 2 receives notification of the urine level and/or the urination timing, the processing device 2 causes the notifier 24 to operate. For example, the processing device 2 turns on an LED lamp as the notifier 24 in accordance with the urine level. In a case where a plurality of LED lamps are provided in association with urine levels, the processing device 2 turns on one of the LED lamps in accordance with the current urine level. In a case where one LED lamp is provided, the processing device 2 changes the illumination mode in accordance with the urine level, such as accelerates the blinking speed in accordance with the urine level. In a case where the processing device 2 receives notification of the urination timing, the processing device 2 turns on the LED lamp in an illumination mode associated with the timing. When the user terminal 71 receives notification of the urine level and/or the urination timing, the user terminal 71 shows notification of the urine level and/or the urination timing on a display of the user terminal 71.

Accordingly, the subject and/or a person near the subject is capable of knowing the urine level and the urination timing and preparing for urination. The subject is capable of preparing for the lavatory early. A person near the subject is also capable of guiding the subject to the lavatory early. Consequently, incontinence can be avoided. Alternatively, in a case where actual urination occurs earlier than expected or preparation for urination takes time, incontinence might occur. Even in such a case, however, a treatment after incontinence can be taken early. That is, the time for exchanging diapers can be notified early.

Through the foregoing processes, the urine volume estimator 52 estimates the urine level, and notifies the subject or a person near the subject of the urine level and/or the urination timing.

In the foregoing description, the urine level is transmitted to the processing device 2 and the user terminal 71 based on arrival of the notification timing. However, the present disclosure is not limited to this example. For example, the urine volume estimator 52 only needs to determine the urine level and cause the third server 33 to store determination results of the urine level, and may not perform notification of the urine level. That is, the subroutine of FIG. 10 may finish at step Sc3 and the process may return to the main flowchart of FIG. 9. Thereafter, if the second server 32 receives a request from the processing device 2 or the user terminal 71, the second server 32 may perform a process similar to step Sc5 and return a urine level and/or a urination timing to the processing device 2 or the user terminal 71.

<Contact Determination

Next, contact determination will be specifically described. FIG. 17 is a flowchart of a subroutine of contact determination.

In step Sd1, the calculator 54 reads the first latest received signals of the ultrasonic sensor 11, the second latest (i.e., one time before) received signals of the ultrasonic sensor 11, and the third latest (i.e., two times before) received signals from the third server 33. Since the probe 1 includes the four ultrasonic sensors 11, the received signals of the ultrasonic sensors 11 to be read out refer to a set of four received signals. That is, the urine volume estimator 52 reads the three sets of received signals from the third server 33.

Thereafter, in step Sd2, the calculator 54 performs standardization and dimension reduction of received signals. The standardization and dimension reduction are performed on each of the first latest received signals, the second latest received signals, and the third latest received signals, and also performed on each of four received signals included in each of the first through third latest received signals.

Specifically, the received signals are constituted by a predetermined number of numerical values arranged in chronological order. Ultrasonic waves propagating in the body attenuate in accordance with the propagation distance. Thus, in received signals, values of reflected waves from portions close to the body surface, that is, temporally early regions, tend to be relatively large, and values of reflected waves from portions far from the body surface, that is, temporally late regions, tend to be relatively small. In view of this, the calculator 54 standardizes received signals to thereby reduce the influence of the propagation distance on numerical values. Accordingly, subsequent calculation of similarities can be appropriately performed. Note that the storage 44 stores mean values and standard deviations of received signals, and the calculator 54 standardizes numerical values of received signals at each time by using these mean values and standard deviations. The storage 44 may store previously acquired mean values and standard deviations of received signals or may store mean values and standard deviations calculated each time based on received signals transmitted from the processing device 2.

Thereafter, the calculator 54 performs dimension reduction on standardized received signals. For example, the calculator 54 performs dimension reduction such as principal component analysis (PCA) or singular value decomposition (SVD). Instead of dimension reduction such as principal component analysis, the calculator 54 may reduce a numerical value of a region having a long propagation distance, that is, a numerical value of a late time, among received signals. The numerical value at a late time has a small value and small amount of information, and thus, the influence of reduction of the numerical value on the similarities is small.

Subsequently, in step Sd3, the calculator 54 calculates similarities of received signals. In this embodiment, for each of the four received signals of the ultrasonic sensors 11, the calculator 54 calculates a similarity between the first latest received signal and the second latest received signal, and a similarity between the second latest received signal and the third latest received signal. That is, the calculator 54 calculates a similarity between the first latest received signal and the second latest received signal regarding the first ultrasonic sensor 11A, a similarity between the first latest received signal and the second latest received signal regarding the second ultrasonic sensor 11B, a similarity between the first latest received signal and the second latest received signal regarding the third ultrasonic sensor 11C, and a similarity between the first latest received signal and the second latest received signal regarding the fourth ultrasonic sensor 11D. The same holds for similarities between the second latest received signals and the third latest received signals.

In this example, the calculator 54 uses a similarity S represented by Equation (1) below.

$$\text{Similarity } S = -1 \times \log(1 - \text{cosine similarity}) \qquad (1)$$

where log is a common logarithm. Supposing that each received signal is assumed as a vector, the cosine similarity is represented by Equation (2) below:

[Equation 1]

$$\text{cosine similarity} = \frac{\vec{q} \cdot \vec{d}}{|\vec{q}| \times |\vec{d}|} \quad (2)$$

The calculator 54 performs smoothing on similarities S between first latest received signals and second latest received signals and similarities S between second latest received signals and third latest received signals. In this embodiment, the calculator 54 calculates an arithmetic mean of the similarities S. In step Sd3, the calculator 54 calculates the smoothed similarities S as final similarities S. As described above, since the received signals include received signals of the four ultrasonic sensors 11, four smoothed similarities S associated with the received signals of the four ultrasonic sensors 11 are calculated. The calculator 54 causes the third server 33 to store similarities S. Step Sd3 is an example of comparing and calculating.

Once similarities S are calculated, the determiner 55 determines whether the similarities S are greater than or equal to a determination threshold or not in step Sd4. The determiner 55 reads the determination threshold 83 from the storage 44. The determiner 55 determines a similarity S for each of the four ultrasonic sensors 11. If the similarity S of at least one of the four ultrasonic sensors 11 is greater than or equal to the determination threshold, the determiner 55 determines that the contact state of the probe 1 with the body surface is inappropriate (step Sd5). On the other hand, if the similarities S of all the four ultrasonic sensors 11 are smaller than the determination threshold, the determiner 55 determines that the contact state of the probe 1 with the body surface is appropriate (step Sd6). The determiner 55 stores the determination result in the third server 33. Step Sd4 is an example of determining.

If the contact state of the probe 1 with the body surface is appropriate, the ultrasonic sensors 11 appropriately transmits ultrasonic waves into the body, and appropriately receives ultrasonic waves from the body. In the body, organs such as small intestine and large intestine function. The body movement, respiration, and pulsation, for example, of the subject can also affect received waves. Thus, if received waves of the ultrasonic sensors 11 are acquired at different times, the shapes of these received waves rarely coincide with one another. If the contact state of the probe 1 is appropriate, these influences can appear in received signals. In this case, when two received signals acquired at different times are compared, a difference appears between these two received signals. That is, the two received signals are not similar to each other, and the similarity S is small.

On the other hand, in a case where the contact state of the probe 1 is inappropriate, such as a case where a gap is present between the probe 1 and the body surface, the ultrasonic sensors 11 can neither appropriately transmit ultrasonic waves into the body nor appropriately receive ultrasonic waves from the inside of the body. Received signals in this case do not include reflected waves from organs and, in addition, do not show movement of the organs and body motion, for example. In this case, a comparison between two received signals acquired at different times shows substantially no difference, that is, the two received signals are closely similar to each other, and the similarity S is large. In a case where the probe 1 is partially separated from the body surface, for example, some of the ultrasonic sensors 11 can appropriately transmit and receive ultrasonic waves, and the other ultrasonic sensors 11 cannot appropriately transmit and receive ultrasonic waves in some cases.

The determination threshold is set at a similarity S at which it can be evaluated that the probe 1 is not in appropriate contact with the body surface. Thus, if the similarity S is smaller than the determination threshold, it is determined that the contact state of the probe 1 with the body surface is appropriate. In contrast, if the similarity S is greater than or equal to the determination threshold, it is determined that the contact state of the probe 1 with the body surface is inappropriate.

If the similarities S of all the ultrasonic sensors 11 are smaller than the determination threshold (step Sd6), the determiner 55 finishes contact determination, and the process returns to the flowchart of FIG. 9 (specifically, step Sb6).

On the other hand, if the similarity S of at least one of the ultrasonic sensors 11 is greater than or equal to the determination threshold (step Sd5), the determiner 55 notifies the processing device 2 and the user terminal 71 that the contact state of the probe 1 is inappropriate through the communicator 43. At this time, the determiner 55 may additionally notify the ultrasonic sensor 11 whose similarity S is greater than or equal to the determination threshold. In addition, the determiner 55 causes the storage 44 to store, as inappropriate received signals, the first latest received signals, the second latest received signals, and the third latest received signals that are a basis of calculation of the similarity S. Thereafter, the determiner 55 finishes contact determination, and the process returns to the flowchart of FIG. 9 (specifically, step Sb6).

When the processing device 2 receives a notification of inappropriate contact state of the probe 1, the processing device 2 operates the notifier 24 in an associated mode. When the user terminal 71 receives a notification of inappropriate contact state of the probe 1, the user terminal 71 provides an associated display. In this manner, the subject or a person near the subject can be suggested to modify the state of attachment of the probe 1.

In the case where a corresponding received signal is stored as an inappropriate received signal in reading received signals in step Sc1 described above, the urine volume estimator 52 does not use these received signals in processes subsequent to step Sc2. In this manner, the urine volume can be more accurately estimated.

In the foregoing description, if the contact state of the probe 1 is inappropriate, notification of this is transmitted to the processing device 2 and the user terminal 71, but the present disclosure is not limited to this. For example, the contact determiner 53 only needs to determine the contact state of the probe 1 and cause the third server 33 to store the determination result, and may not issue notification of an inappropriate contact state. That is, when the second server 32 receives a request from the processing device 2 or the user terminal 71, the second server 32 may perform a process similar to step Sd5 and return the contact state of the probe 1 to the processing device 2 or the user terminal 71.

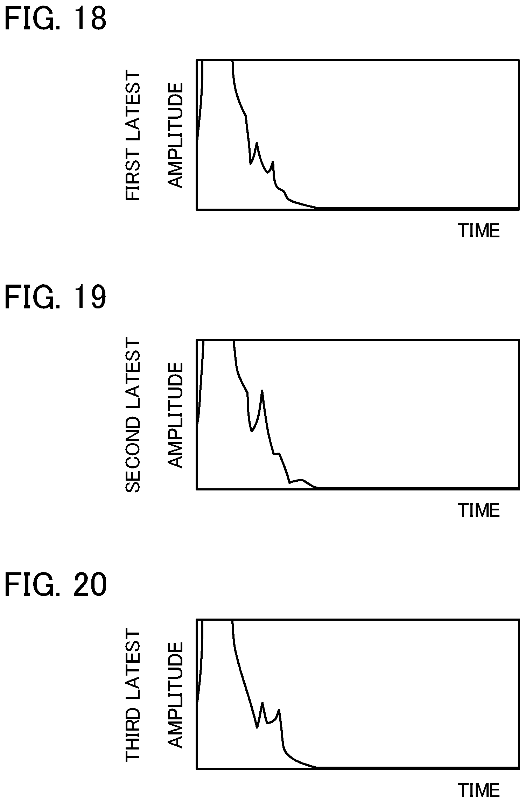

FIGS. 18 through 20 show, as an example, received signals of the third ultrasonic sensor 11C in the case where the contact state of the probe 1 with the body surface is appropriate. FIG. 18 shows a first latest received signal. FIG. 19 shows a second latest signal waveform. FIG. 20 shows a third latest received waveform. The received signals of FIGS. 18 through 20 are signals subjected to the averaging process by the processing device 2. A comparison among FIGS. 18 through 20 shows a large difference in waveform, and the three waveforms are not similar to one another. The similarity S between the received signal of FIG. 18 and the received signal of FIG. 19 is 2.0. The similarity S between the received signal of FIG. 19 and the received signal of FIG. 20 is 2.1. The mean value of the two similarities S is 2.05. For example, the determination threshold is set at 3.5. The mean value of 2.05 is smaller than the determination threshold, and thus, it is determined that the contact state of the probe 1 is appropriate.

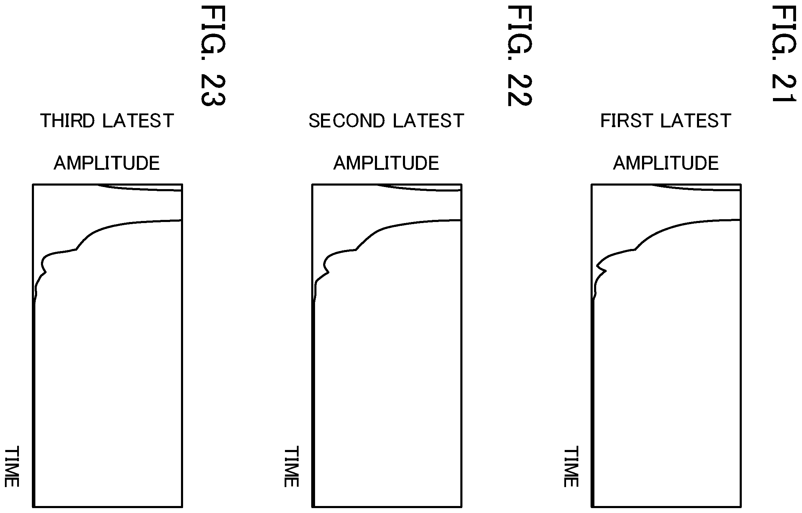

FIGS. 21 through 23 show, as an example, received signals of the third ultrasonic sensor 11C in the case where the contact state of the probe 1 with the body surface is inappropriate. FIG. 21 shows a first latest received signal. FIG. 22 shows a second latest received waveform. FIG. 23 shows a third latest received waveform. The received signals of FIGS. 21 through 23 are signals subjected to the averaging process by the processing device 2. A comparison among FIGS. 21 through 23 shows a small difference in waveform, and the three waveforms are similar to one another. The similarity S between the received signal of FIG. 21 and the received signal of FIG. 22 is 4.4. The similarity S between the received signal of FIG. 21 and the received signal of FIG. 22 is 4.5. The mean value of the two similarities S is 4.45. The mean value of 4.45 is larger than the determination threshold, and thus, it is determined that the contact state of the probe 1 is inappropriate.

As described above, the ultrasonic measurement device 100 includes: the probe 1 configured to be attached to the body surface while being in contact with the body surface, transmit ultrasonic waves into the body, and receive reflected waves of ultrasonic waves; and the controller 41 configured to detect a contact state of the probe 1 with the body surface. The controller 41 determines the contact state of the probe 1 with the body surface by comparing at least two received waves, that is, received signals, acquired through transmission and reception of ultrasonic waves by the probe 1 at different times.

The first server 31 is a contact determination server device that is communicable, through the network, with the terminal device 10 including the probe 1 configured to be attached to the body surface while being in contact with the body surface, transmit ultrasonic waves into the body, and receive reflected waves of ultrasonic waves. The first server 31 receives received waves, that is, received signals, transmitted from the terminal device 10, and includes the controller 41 configured to determine a contact state of the probe 1 with the body surface. The controller 41 determines the contact state of the probe 1 with the body surface by comparing at least two received signals acquired through transmission and reception of ultrasonic waves by the probe 1 at different times.

In addition, the contact determination program 82 is a contact determination program that causes the first server 31 (computer) to execute a function of determining a contact state of the probe 1 with a body surface of a subject, and the probe 1 is configured to be attached to the body surface while being in contact with the body surface, transmit ultrasonic waves into a body of the subject, and receive reflected waves of ultrasonic waves. The contact determination program 82 is configured to cause the first server 31 to execute: a comparison function of comparing at least two received waves, that is, received signals, acquired through transmission and reception of ultrasonic waves by the probe 1 at different times; and a determination function of determining a contact state of the probe 1 with the body surface based on a result of the comparison function.

In addition, a contact determination method of the ultrasonic measurement device 100 is a contact determination method for determining a contact state of the probe 1 with the body surface of a subject, and the probe 1 is configured to be attached to the body surface while being in contact with the body surface, transmit ultrasonic waves into the body, and receive reflected waves of ultrasonic waves. The method includes step Sd3 (comparing) of comparing at least two received waves, that is, received signals, acquired through transmission and reception of ultrasonic waves by the probe 1 at different times and step Sd4 (determining) of determining a contact state of the probe 1 with the body surface based on a result of the comparing.

With these configurations, the contact state of the probe 1 with the body surface can be determined as long as two received signals can be acquired through transmission and reception of ultrasonic waves at different times. Specifically, in the case where the probe 1 is appropriately in contact with the body surface, the probe 1 appropriately transmits ultrasonic waves into the body, and appropriately receives reflected waves from the inside of the body with the probe 1. Received waves including reflected waves from the inside of the body are under the influence of movement of organs and body motion, for example. Since the movement of organs and the body motion, for example, are irregular, if received signals in transmitting and receiving ultrasonic waves at different timing are compared, these two received signals are expected to show a change, that is, a difference. On the other hand, if the probe 1 is not appropriately in contact with the body surface, two received signal does not show a significant change. Thus, by comparing two received signals acquired at different times, a contact state of the probe 1 with the body surface can be determined. As described above, since the contact state of the probe 1 with the body surface can be determined by comparing two received signals acquired at different times by the probe 1, the contact state of the probe 1 can be easily determined without any work such as observation of received signals while adjusting the contact state of the probe 1. Since the contact state of the probe 1 can be determined with the probe 1 being kept attached to the body surface, the contact state of the probe 1 can be determined while an originally intended process (urine volume estimation in the above example) by the probe 1 is performed or a state where the originally intended process is capable of being performed is maintained. In particular, this embodiment is effective for the case of using the probe 1 while continuously attaching the probe 1 to the body.

Specifically, the controller 41 determines the contact state of the probe 1 with the body surface based on the degree of similarity between at least two received waves, that is, received signals.

In the contact determination program 82, the comparison function is a calculation function of calculating the degree of similarity between at least two received waves, that is, received signals, and the determination function determines the contact state of the probe 1 with the body surface based on the calculated degree of similarity.

In addition, in the contact determination method of the ultrasonic measurement device 100, the comparing is calculating the degree of similarity between at least two received waves, that is, received signals, and in the determining, the contact state of the probe 1 with the body surface is determined based on the calculated degree of similarity.

With these configurations, the contact state of the probe 1 can be determined using a constant criterion. That is, as described above, in the case where contact state of the probe 1 with the body surface is appropriate, two received signals show a difference, whereas in the case where the contact state of the probe 1 with the body surface is inappropriate, the two received signals hardly show a difference. Such a difference between two received signals can be evaluated by various method. For example, the degree of similarity between two received signals clearly shows a difference between the two received signals. In view of this, in comparing two received signals, the controller 41 determines the contact state of the probe 1 using a criterion of the degree of similarity between two received signals. Accordingly, the contact state of the probe 1 can be determined at a constant criterion.

More specifically, the controller 41 determines the contact state of the probe 1 with the body surface based on the similarity S between at least two received waves, that is, two received signals.

The calculation function of the contact determination program 82 calculates the similarity S between at least two received waves, that is, two received signals.

In the contact determination method of the ultrasonic measurement device 100, in the calculating, the similarity S between at least two received waves, that is, two received signals, is calculated.

With these configurations, by using the parameter of the similarity S, the contact state of the probe 1 can be uniformly determined. The similarity S is associated with a cosine similarity between two received signals. Thus, it is determined whether two received signals are similar to each other as a whole. For example, the degree of similarity between two received signals can also be evaluated by using local features such as the presence/absence of a peak, the magnitude of the peak, or a position (i.e., the time) of the peak. On the other hand, the cosine similarity represents the degree of similarity between two vectors, where two received signals are regarded as two vectors. That is, the cosine similarity represents a total degree of similarity between two received signals. Thus, it is possible to determine whether two received signals are similar to each other as a whole by evaluating the degree of similarity between two received signals based on the similarity S represented by using the cosine similarity.

The contact determination method of the ultrasonic measurement device 100 further includes an acquisition step of acquiring at least two received waves, that is, received signals, through transmission and reception of ultrasonic waves by the probe 1 at different times.

«Other Embodiments»

In the foregoing section, the embodiment has been described as an example of the technique disclosed in the present application. The technique disclosed here, however, is not limited to this embodiment, and is applicable to other embodiments obtained by changes, replacements, additions, and/or omissions as necessary. Components described in the above embodiment may be combined as a new exemplary embodiment. Components provided in the accompanying drawings and the detailed description can include components unnecessary for solving problems as well as components necessary for solving problems in order to exemplify the technique. Therefore, it should not be concluded that such unnecessary components are necessary only because these unnecessary components are included in the accompanying drawings or the detailed description.

The above embodiment may be configured as described below.

Although the probe 1 is used for detecting the urinary bladder, the target of detection by the probe 1 is not limited to the urinary bladder. That is, the ultrasonic measurement device 100 has a main object of estimating a urine volume in the urinary bladder, but the main object, that is, a basic process, of the ultrasonic measurement device 100 is not limited to estimation of the urine volume. That is, the contact determination described above is applicable to a device and a method in which ultrasonic waves are transmitted into the body from the probe 1 configured to be attached to the body surface of a subject while being in contact with the body surface, reflected waves of ultrasonic waves are received by the probe 1, and any process is performed based on the received signal.

The probe 1 may be placed at any position on the body surface. The method for attaching the probe 1 is not limited to the method described above. For example, the contact surface 13 may be an adhesive surface so that the contact surface 13 can be attached to the abdomen of the subject.

The number of ultrasonic sensors 11 is not limited to four. The number of ultrasonic sensors 11 may be one, or three or less, or five or more.

The arrangement of the plurality of ultrasonic sensors 11 is not limited to the arrangement described above. For example, the ultrasonic sensors 11 may not be offset in the lateral direction.

The casing 12 is not limited to the configuration described above. For example, a projection may be provided on the contact surface 13 of the casing 12. The ultrasonic sensor 11 is incorporated in the projection. The projection increases adhesion of a portion of the casing 12 including the ultrasonic sensor 11 with the skin (body surface) so that entering of ultrasonic waves into the human body is promoted. Accordingly, detection capacity of the urinary bladder can be enhanced.

Although the probe 1 and the processing device 2 are configured as separate components, the present disclosure is not limited to this example. For example, the probe 1 and the processing device 2 may be configured as one unit. The probe 1 and the processing device 2 may not be connected by wires, and may communicate with each other wirelessly. In addition, the probe 1 has a part of the function (e.g., the transmitter 21 or the receiver 22) of the processing device 2.

The configuration of the processing device 2 is not limited to the configuration described above. For example, although the transmitter 21 inputs a pulse signal into the probe 1 as a driving signal, the driving signal is not limited to the pulse signal. The driving signal may not be, for example, a burst wave, instead of a pulse wave. The notifier 24 is not limited to an LED lamp, and may be a display, an alarm, or a vibrator.

The processing device 2 is wirelessly connected to the server group 3, more specifically, the first server 31, but may be connected by wires.

The processes of the processing device 2 and the server group 3 described above are not unique to these devices, and at least a part of the processes may be executed by a device different from those described above. For example, estimation of the urine volume may be performed by the processing device 2. Alternatively, the ultrasonic measurement device 100 may not include the server group 3, and the function of the server group 3 described above may be implemented by the processing device 2 or another device (e.g., a PC or a user terminal 71).

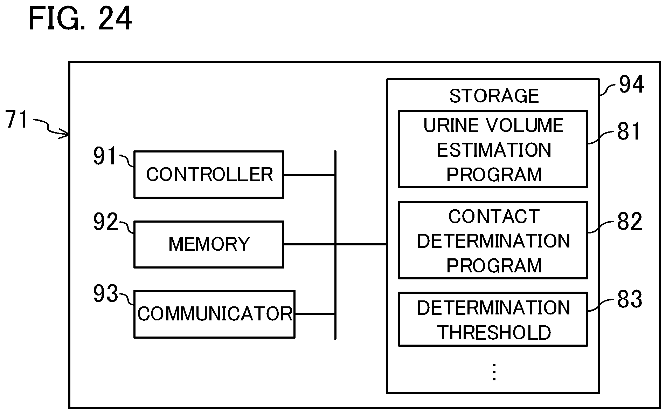

For example, the user terminal 71 may have the function of the server group 3. FIG. 24 is a block diagram illustrating a hardware configuration of the user terminal 71. In the case where the user terminal 71 has the function of the server group 3, the communicator 93 of the user terminal 71 communicates with the processing device 2 wirelessly or by wires. The controller 91 of the user terminal 71 functions as the controller 41 of the first server 31. The storage 94 stores the urine volume estimation program 81, the contact determination program 82, and the determination threshold 83. In addition, the storage 94 also serves as the third server 33, and stores received signals of the ultrasonic sensors 11 and determination results, for example. The controller 91 functions as the acquirer 51, the urine volume estimator 52, and the contact determiner 53 by developing programs stored in the storage 94 to the memory 92 and executing the developed programs. That is, the controller 91 has a function configuration similar to that of the controller 41 illustrated in FIG. 8. In this case, the user terminal 71 functions as a contact determination server device.

Although the server group 3 includes a plurality of servers, one server may have the functions of the first through third servers 31 through 33.

The operation of the ultrasonic measurement device 100 described above is merely an example. For example, detection of reflected waves by the ultrasonic sensors 11 and determination on whether the urine level changes or not are performed periodically, but the present disclosure is not limited to this example. For example, the ultrasonic measurement device 100 may perform detection of reflected waves by the ultrasonic sensors 11 and determination on whether the urine level changes or not when the subject is in a predetermined posture. The shape and position of the urinary bladder in the body can change depending on the posture of the subject. Thus, the posture of the subject is made uniform in transmitting and detecting ultrasonic waves so that the urinary bladder can be accurately detected, and the urine level can be accurately determined. Alternatively, the ultrasonic measurement device 100 may determine whether the urine level changes or not by using a reflected wave detected at the predetermined posture among reflected waves periodically detected by the ultrasonic sensors 11. Alternatively, the ultrasonic measurement device 100 may be configured to urge the subject to take a predetermined posture in detecting reflected waves by the ultrasonic sensors 11.

Although the ultrasonic measurement device 100 determines the urine level based on to which one of the ultrasonic sensors 11 detects the urinary bladder, the present disclosure is not limited to this example. The estimation of the urine volume can be performed by any method.

The user terminal 71 is registered in the server group 3, and a determination result of the server group 3, for example, is transmitted to the user terminal 71 when necessary. Alternatively, the user terminal 71 may not be registered. In this case, the process to the user terminal 71 is omitted in the processes described above.

The contact determination is not limited to the method described above. For example, the controller 41 compares two received signals acquired at different times, and evaluates a difference between the two received signals based on at least one of the presence/absence, magnitude, and position (received time) of a peak (reflected wave) included in the received signals to thereby determine the contact state of the probe 1.

The index representing the degree of similarity may be any index as long as the index represents the degree of similarity between two received signals. For example, the cosine similarity may be used as it is, as a similarity representing the degree of similarity between two received signals. Alternatively, an inverse number of a Euclidean distance or a Pearson moment correlation coefficient, for example, may be used as a similarity.

The process on received signals before calculation of the similarity S is not necessary. That is, standardization and dimension reduction of received signals may be omitted.

In addition, although a plurality of similarities S are calculated and then smoothed (averaged), the present disclosure is not limited to this example. The controller 41 may calculate a similarity S from only two received signals so that this similarity S can be used for determination of the contact state of the probe 1. In the case of smoothing of similarities S, the number of similarities S for the smoothing is not limited to two. That is, the controller 41 may calculate three or more similarities S by using four or more received signals and smooth these similarities S. In a case where a large number of similarities S are smoothed, a trimmed mean or a Winsorized mean may be used instead of an arithmetic mean. The technique for smoothing is not limited to averaging, and may be a technique using a Gaussian filter or a spline interpolation.

The similarity S is not limited to a similarity S between two received signals that are chronologically successive. For example, in the example described above, the controller 41 may calculate a similarity S between the first latest received signal and the third latest received signal. That is, two received signals used for calculating a similarity S may be two received signals that are not chronologically successive and acquired with a predetermined time interval.

INDUSTRIAL APPLICABILITY

As described above, the technique disclosed here is useful for an ultrasonic measurement device, a contact determination server device, a contact determination program, and a contact determination method.

DESCRIPTION OF REFERENCE CHARACTERS

100 ultrasonic measurement device
1 probe
10 terminal device
31 first server (contact determination server device)
41 controller

**Claims**

1. A contact determination server device (31) communicable, through a network, with a terminal device (10) including a probe (1), the probe (1) being configured to be continuously attached to a body surface of a subject while being in contact with the body surface, transmit ultrasonic waves into a body of the subject, and receive reflected waves of ultrasonic waves, the contact determination server device being configured to receive reflected waves transmitted from the terminal device (10), the contact determination server device (31) comprising

a controller (41) configured to determine a contact state of the probe (1) with the body surface and determine whether a urinary bladder (64) is detected or not based on received waves, wherein

the controller (41) determines the contact state of the probe (1) with the body surface by comparing at least two received waves acquired through transmission and reception of ultrasonic waves by the probe (1) at different times of the received waves obtained to determine whether the urinary bladder (64) is detected or not.

2. The contact determination server device (31) according to claim 1, **characterized in that**
the controller (41) determines the contact state of the probe (1) with the body surface based on a degree of similarity between the at least two received waves.

3. An ultrasonic measurement device (100) comprising:
a probe (1) configured to be attached to a body surface of a subject while being in contact with the body surface, transmit ultrasonic waves into a body of the subject, and receive reflected waves of ultrasonic waves; and a contact determination service (31) device according to claim 1 or 2.

4. A contact determination program (82) configured to cause a computer to execute a function of determining a contact state of a probe (1) with a body surface of a subject, the probe (1) being configured to be continuously attached to the body surface while being in contact with the body surface, transmit ultrasonic waves into a body of the subject, and receive reflected waves of ultrasonic waves, the contact determination program (82) being configured to cause the computer to execute:

a detection function of determining whether a urinary bladder (64) is detected or not based on received waves;
a comparison function of comparing at least two received waves acquired through transmission and reception of ultrasonic waves by the probe at different times of the received waves obtained to determine whether the urinary bladder (64) is detected or not; and
a determination function of determining the contact state of the probe (1) with the body surface based on a result of the comparison function.

5. The contact determination program (82) according to claim 4, **characterized in that**

the comparison function is a calculation function of calculating a degree of similarity between the at least two received waves, and
the determination function determines the contact state of the probe (1) with the body surface based on the calculated degree of similarity.

6. A contact determination method for determining a contact state of a probe (1) with a body surface of a subject, the probe (1) being configured to be continuously attached to the body surface while being in contact with the body surface, transmit ultrasonic waves into a body of the subject, and receive reflected waves of ultrasonic waves, the method comprising the steps of:

determining whether a urinary bladder (64) is detected or not based on received waves;
comparing at least two received waves acquired through transmission and reception of ultrasonic waves by the probe at different times of the received waves obtained to determine whether the urinary bladder (64) is detected or not; and
determining the contact state of the probe (1) with the body surface based on a result of the comparing.

7. The contact determination method according to claim 6, **characterized by** further comprising the step of:
acquiring at least two received waves through transmission and reception of ultrasonic waves by the probe at different times.

8. The contact determination method according to claim 6 or 7, wherein

the comparing is calculating a degree of similarity between the at least two received waves, and
in the determining, the contact state of the probe with the body surface is determined based on the calculated degree of similarity.

**Patentansprüche**

1. Kontaktbestimmungsservervorrichtung (31), die über ein Netzwerk mit einem Endgerät (10), das eine Sonde (1) enthält, kommunizieren kann, wobei die Sonde (1) konfiguriert ist, um kontinuierlich an einer Körperoberfläche eines Subjekts angebracht zu sein, während sie in Kontakt mit der Körperoberfläche steht, Ultraschallwellen in einen Körper des Subjekts zu senden und reflektierte Wellen von Ultraschallwellen zu empfangen, wobei die Kontaktbestimmungsservervorrichtung konfiguriert ist, um von dem Endgerät (10) gesendete reflektierte Wellen zu empfangen, wobei die Kontaktbestimmungsservervorrichtung (31) folgendes umfasst:

eine Steuerung (41), die konfiguriert ist, um einen Kontaktzustand der Sonde (1) mit der Körperoberfläche zu bestimmen und auf der Grundlage der empfangenen Wellen festzustellen, ob eine Harnblase (64) erkannt wird oder nicht, wobei die Steuerung (41) den Kontaktzustand der Sonde (1) mit der Körperoberfläche bestimmt, indem sie mindestens zwei empfangene Wellen miteinander vergleicht, die durch Senden und Empfangen von Ultraschallwellen durch die Sonde (1) zu unterschiedlichen Zeitpunkten der empfangenen Wellen erhalten wurden, um zu bestimmen, ob die Harnblase (64) erkannt wird oder nicht.

2. Kontaktbestimmungsservervorrichtung (31) nach Anspruch 1, **dadurch gekennzeichnet, dass** die Steuerung (41) den Kontaktzustand der Sonde (1) mit der Körperoberfläche auf der Grundlage eines Grades der Ähnlichkeit zwischen den mindestens zwei empfangenen Wellen bestimmt.

3. Ultraschallmessgerät (100), umfassend:

eine Sonde (1), die konfiguriert ist, um an einer Körperoberfläche eines Subjekts angebracht zu sein, während sie in Kontakt mit der Körperoberfläche steht, Ultraschallwellen in einen Körper des Subjekts zu senden und reflektierte Wellen von Ultraschallwellen zu empfangen; und
eine Kontaktbestimmungsservervorrichtung (31) nach Anspruch 1 oder 2.

4. Kontaktbestimmungsprogramm (82), das konfiguriert ist, um einen Computer zu veranlassen, eine Funktion zur Bestimmung eines Kontaktzustands einer Sonde (1) mit einer Körperoberfläche eines Subjekts auszuführen, wobei die Sonde (1) konfiguriert ist, um kontinuierlich an der Körperoberfläche angebracht zu sein, während sie in Kontakt mit der Körperoberfläche steht, Ultraschallwellen in einen Körper des Subjekts zu senden und reflektierte Wellen von Ultraschallwellen zu empfangen, wobei das Kontaktbestimmungsprogramm (82) konfiguriert ist, um den Computer zu veranlassen, folgendes auszuführen:

eine Erkennungsfunktion, die auf der Grundlage der empfangenen Wellen bestimmt, ob eine Harnblase (64) erkannt wird oder nicht;
eine Vergleichsfunktion, die mindestens zwei empfangene Wellen miteinander vergleicht, die durch Senden und Empfangen von Ultraschallwellen durch die Sonde zu unterschiedlichen Zeitpunkten der empfangenen Wellen erhalten wurden, um zu bestimmen, ob die Harnblase (64) erkannt wird oder nicht; und
eine Bestimmungsfunktion zur Bestimmung des Kontaktzustandes der Sonde (1) mit der Körperoberfläche auf der Grundlage eines Ergebnisses der Vergleichsfunktion.

5. Kontaktbestimmungsprogramm (82) nach Anspruch 4, **dadurch gekennzeichnet, dass** die Vergleichsfunktion eine Berechnungsfunktion zur Berechnung eines Grades der Ähnlichkeit zwischen den mindestens zwei empfangenen Wellen ist, und
die Bestimmungsfunktion den Kontaktzustand der Sonde (1) mit der Körperoberfläche auf der Grundlage des berechneten Grades der Ähnlichkeit bestimmt.

6. Kontaktbestimmungsverfahren zur Bestimmung eines Kontaktzustands einer Sonde (1) mit einer Körperoberfläche eines Subjekts, wobei die Sonde (1) konfiguriert ist, um kontinuierlich an der Körperoberfläche angebracht zu sein, während sie in Kontakt mit der Körperoberfläche steht, Ultraschallwellen in einen Körper des Subjekts zu senden und reflektierte Wellen von Ultraschallwellen zu empfangen, wobei das Verfahren die folgenden Schritte umfasst:

Bestimmen, ob eine Harnblase (64) erkannt wird oder nicht, auf der Grundlage der empfangenen Wellen;
Vergleichen von mindestens zwei empfangenen Wellen miteinander, die durch Senden und Empfangen von Ultraschallwellen durch die Sonde zu unterschiedlichen Zeitpunkten der empfangenen Wellen erhalten wurden, um zu bestimmen, ob die Harnblase (64) erkannt wird oder nicht; und
Bestimmen des Kontaktzustandes der Sonde (1) mit der Körperoberfläche auf der Grundlage des Vergleichs.

7. Kontaktbestimmungsverfahren nach Anspruch 6, **dadurch gekennzeichnet, dass** es des Weiteren den folgenden Schritt umfasst:
Erfassen von mindestens zwei empfangenen Wellen durch Senden und Empfangen von Ultraschallwellen durch die Sonde zu unterschiedlichen Zeitpunkten.

**8.** Kontaktbestimmungsverfahren nach Anspruch 6 oder 7, wobei bei dem Schritt des

Vergleichens eine Berechnung eines Grades der Ähnlichkeit zwischen den mindestens zwei empfangenen Wellen erfolgt, und
bei dem Schritt des Bestimmens der Kontaktzustand der Sonde mit der Körperoberfläche auf der Grundlage des berechneten Grades der Ähnlichkeit bestimmt wird.

**Revendications**

**1.** Dispositif serveur de détermination de contact (31) apte à communiquer, par le biais d'un réseau, avec un dispositif terminal (10) incluant une sonde (1), la sonde (1) étant configurée pour être constamment attachée à une surface corporelle d'un sujet tout en étant en contact avec la surface corporelle, émettre des ondes ultrasonores dans un corps du sujet, et recevoir des ondes réfléchies d'ondes ultrasonores, le dispositif serveur de détermination de contact étant configuré pour recevoir des ondes réfléchies émises par le dispositif terminal (10), le dispositif serveur de détermination de contact (31) comprenant

un dispositif de commande (41) configuré pour déterminer un état de contact de la sonde (1) avec la surface corporelle et déterminer si une vessie urinaire (64) est détectée ou non sur la base d'ondes reçues, dans lequel le dispositif de commande (41) détermine l'état de contact de la sonde (1) avec la surface corporelle par la comparaison d'au moins deux ondes reçues acquises par le biais de l'émission et de la réception d'ondes ultrasonores par la sonde (1) à différents temps des ondes reçues obtenues pour déterminer si la vessie urinaire (64) est détectée ou non.

**2.** Dispositif serveur de détermination de contact (31) selon la revendication 1, **caractérisé en ce que** le dispositif de commande (41) détermine l'état de contact de la sonde (1) avec la surface corporelle sur la base d'un degré de similarité entre les au moins deux ondes reçues.

**3.** Dispositif de mesure ultrasonore (100) comprenant:

une sonde (1) configurée pour être attachée à une surface corporelle d'un sujet tout en étant en contact avec la surface corporelle, émettre des ondes ultrasonores dans un corps du sujet,
et recevoir des ondes réfléchies d'ondes ultrasonores; et un dispositif de service de détermination de contact (31) selon la revendication 1 ou 2.

**4.** Programme de détermination de contact (82) configuré pour amener un ordinateur à exécuter une fonction de détermination d'un état de contact d'une sonde (1) avec une surface corporelle d'un sujet, la sonde (1) étant configurée pour être constamment attachée à la surface corporelle tout en étant en contact avec la surface corporelle, émettre des ondes ultrasonores dans un corps du sujet, et recevoir des ondes réfléchies d'ondes ultrasonores, le programme de détermination de contact (82) étant configuré pour amener l'ordinateur à exécuter:

une fonction de détection consistant à déterminer si une vessie urinaire (64) est détectée ou non sur la base d'ondes reçues;
une fonction de comparaison consistant à comparer au moins deux ondes reçues acquises par le biais de l'émission et de la réception d'ondes ultrasonores par la sonde à différents temps des ondes reçues obtenues pour déterminer si la vessie urinaire (64) est détectée ou non; et
une fonction de détermination consistant à déterminer l'état de contact de la sonde (1) avec la surface corporelle sur la base d'un résultat de la fonction de comparaison.

**5.** Programme de détermination de contact (82) selon la revendication 4, **caractérisé en ce que**

la fonction de comparaison est une fonction de calcul consistant à calculer un degré de similarité entre les au moins deux ondes reçues, et

la fonction de détermination détermine l'état de contact de la sonde (1) avec la surface corporelle sur la base du degré de similarité calculé.

6. Procédé de détermination de contact pour déterminer un état de contact d'une sonde (1) avec une surface corporelle d'un sujet, la sonde (1) étant configurée pour être constamment attachée à la surface corporelle tout en étant en contact avec la surface corporelle, émettre des ondes ultrasonores dans un corps du sujet, et recevoir des ondes réfléchies d'ondes ultrasonores, le procédé comprenant les étapes de:

la détermination de si une vessie urinaire (64) est détectée ou non sur la base d'ondes reçues;
la comparaison d'au moins deux ondes reçues acquises par le biais de l'émission et de la réception d'ondes ultrasonores par la sonde à différents temps des ondes reçues obtenues pour déterminer si la vessie urinaire (64) est détectée ou non; et
la détermination de l'état de contact de la sonde (1) avec la surface corporelle sur la base d'un résultat de la comparaison.

7. Procédé de détermination de contact selon la revendication 6, **caractérisé par le fait qu'**il comprend en outre l'étape de:

l'acquisition d'au moins deux ondes reçues par le biais de l'émission et de la réception d'ondes ultrasonores par la sonde à différents temps.

8. Procédé de détermination de contact selon la revendication 6 ou 7, dans lequel

la comparaison consiste à calculer un degré de similarité entre les au moins deux ondes reçues, et
lors de la détermination, l'état de contact de la sonde avec la surface corporelle est déterminé sur la base du degré de similarité calculé.

FIG. 1

100

3

THIRD
SERVER  33

FIRST
SERVER

32

31

SECOND
SERVER

72

GATEWAY

71

10

PROCESSING
DEVICE

USER
TERMINAL

1

2

PROBE

FIG. 2

1

12

11D

11C

11B

11A

13

FIG. 3

FIG. 4

FIG. 5

FIG. 6

## FIG. 7

31

41 CONTROLLER

42 MEMORY

43 COMMUNICATOR

STORAGE — 44

URINE VOLUME ESTIMATION PROGRAM — 81

CONTACT DETERMINATION PROGRAM — 82

DETERMINATION THRESHOLD — 83

⋮

## FIG. 8

41

53 CONTACT DETERMINER

43 COMMUNICATOR

54 CALCULATOR   55 DETERMINER

52 URINE VOLUME ESTIMATOR

51 ACQUIRER

33 THIRD SERVER

FIG. 9

```
        START                              START

          │                                  │
          ▼                                  ▼
    ┌──────────────┐ Sa1      Sb1 ┌──────────────┐
 NO │ TRANSMISSION/│          ──▶ │ RECEIVE      │ YES
◀───┤  RECEPTION   │              │ RECEIVED     ├────┐
    │   TIMING?    │              │  SIGNAL?     │    │    Sb2
    └──────┬───────┘              └──────┬───────┘    ▼
           │ YES                         │ NO    ┌──────────────┐
           ▼                             │       │ STORE        │
 Sa2 ┌──────────────────────┐           │       │ RECEIVED     │
  ◀──┤ TRANSMIT/RECEIVE BY  │           │       │  SIGNAL      │
     │ FIRST ULTRASONIC     │           │       └──────┬───────┘
     │     SENSOR           │           ◀──────────────┘
     └──────────┬───────────┘           │
                ▼                        ▼
 Sa3 ┌──────────────────────┐ Sb3 ┌──────────────┐
  ◀──┤ TRANSMIT/RECEIVE BY  │  ◀──┤  ESTIMATION  │ NO
     │ SECOND ULTRASONIC    │     │   TIMING?    ├────┐
     │     SENSOR           │     └──────┬───────┘    │
     └──────────┬───────────┘            │ YES   Sb5  ▼
                ▼                         │    ┌──────────────┐
 Sa4 ┌──────────────────────┐            │    │ DETERMINATION│ NO
     │ TRANSMIT/RECEIVE BY  │ Sb4 ┌──────┴─────┐│   TIMING?   ├──▶
     │ THIRD ULTRASONIC     │  ◀──┤ URINE VOLUME│└──────┬──────┘
     │     SENSOR           │     │ ESTIMATION  │       │ YES
     └──────────┬───────────┘     └──────┬──────┘       ▼
                ▼                         │   Sb6 ┌──────────────┐
 Sa5 ┌──────────────────────┐            │    ◀──┤  CONTACT     │
     │ TRANSMIT/RECEIVE BY  │            │       │ DETERMINATION│
     │ FOURTH ULTRASONIC    │            │       └──────┬───────┘
     │     SENSOR           │            ◀──────────────┘
     └──────────┬───────────┘            │
                ▼                         ▼
 Sa6 ┌──────────────┐                   END
  ◀──┤  TRANSMIT    │
     │  RECEIVED    │
     │   SIGNAL     │
     └──────┬───────┘
            ▼
          END
```

EP 3 799 793 B1

## FIG. 10

```
┌──────────────────┐
│  URINE VOLUME    │
│   ESTIMATION     │
└──────────────────┘
          │
          ▼
Sc1 ┌──────────────────┐
    │  READ RECEIVED   │
    │     SIGNAL       │
    └──────────────────┘
          │
          ▼
Sc2 ┌──────────────────┐
    │DETERMINE URINARY │
    │ BLADDER DETECTION│
    └──────────────────┘
          │
          ▼
Sc3 ┌──────────────────┐
    │ DETERMINE URINE  │
    │     LEVEL        │
    └──────────────────┘
          │
          ▼
Sc4    ╱NOTIFICATION╲  NO
       ╲  TIMING?   ╱──────┐
          │ YES            │
          ▼                │
Sc5 ┌─────────────────────┐│
    │NOTIFICATION OF URINE││
    │LEVEL AND URINATION  ││
    │     TIMING          ││
    └─────────────────────┘│
          │                │
          ▼◄───────────────┘
      (  RETURN  )
```

## FIG. 11

FIG. 12

11A

AMPLITUDE

W1

TIME

FIG. 13

11B

AMPLITUDE

W1

TIME

FIG. 14

11C

AMPLITUDE

W2

TIME

FIG. 15

11D

AMPLITUDE

W2

TIME

## FIG. 16

| TIME | 11A | 11B | 11C | 11D | URINE LEVEL |
|---|---|---|---|---|---|
| 8:00 | 0 | 0 | 0 | 0 | 0 |
| 8:10 | 0 | 0 | 0 | 0 | 0 |
| 8:20 | 0 | 0 | 0 | 0 | 0 |
| 8:30 | 1 | 0 | 0 | 0 | 1 |
| 8:40 | 1 | 0 | 0 | 0 | 1 |
| 8:50 | 1 | 0 | 0 | 0 | 1 |
| 9:00 | 1 | 1 | 0 | 0 | 2 |

## FIG. 17

```
        CONTACT
     DETERMINATION

Sd1    READ RECEIVED
          SIGNAL

Sd2   STANDARDIZATION AND
      DIMENSION REDUCTION

Sd3      CALCULATE
         SIMILARITY

Sd4    SIMILARITY ≥           NO
       DETERMINATION
        THRESHOLD ?
           YES                         Sd6
Sd5  CONTACT STATE IS          CONTACT STATE IS
      INAPPROPRIATE              APPROPRIATE

           RETURN
```

FIG. 18

FIRST LATEST

AMPLITUDE

TIME

FIG. 19

SECOND LATEST

AMPLITUDE

TIME

FIG. 20

THIRD LATEST

AMPLITUDE

TIME

FIG. 21

FIRST LATEST

AMPLITUDE

TIME

FIG. 22

SECOND LATEST

AMPLITUDE

TIME

FIG. 23

THIRD LATEST

AMPLITUDE

TIME

## FIG. 24

71

91 — CONTROLLER

92 — MEMORY

93 — COMMUNICATOR

STORAGE — 94

URINE VOLUME ESTIMATION PROGRAM — 81

CONTACT DETERMINATION PROGRAM — 82

DETERMINATION THRESHOLD — 83

**EP 3 799 793 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014037168 A1
- JP 2016043274 A